(19) Europäisches Patentamt
European Patent Office
Office européen des brevets

(11) EP 1 245 249 A2

(12) # DEMANDE DE BREVET EUROPEEN

(43) Date de publication:
**02.10.2002 Bulletin 2002/40**

(21) Numéro de dépôt: **02014143.8**

(22) Date de dépôt: **11.04.1996**

(51) Int Cl.⁷: **A61P 43/00**, A61K 31/23, A61K 39/39, C12N 5/10, C12N 15/88

(84) Etats contractants désignés:
**AT BE CH DE DK ES FI FR GB GR IE IT LI MC NL PT SE**

(30) Priorité: **11.04.1995 FR 9504615**

(62) Numéro(s) de document de la (des) demande(s) initiale(s) en application de l'article 76 CBE:
**96913569.8 / 0 769 942**

(71) Demandeur: **Aventis Pasteur**
**69367 Lyon Cedex 07 (FR)**

(72) Inventeur: **Haensler, Jean**
**69290 Saint Genis Les Olli-res (FR)**

(74) Mandataire: **Kerneis, Danièle et al**
**2, Avenue Pont Pasteur**
**69367 Lyon Cedex 07 (FR)**

Remarques:
Cette demande a été déposée le 25 - 06 - 2002 comme demande divisionnaire de la demande mentionnée sous le code INID 62.

(54) **Utilisation d'un composé amphipathique cationique comme agent de transfection, comme adjuvant de vaccin, ou comme medicament**

(57) L'invention a pour objet un composé amphipathique cationique de formule I :

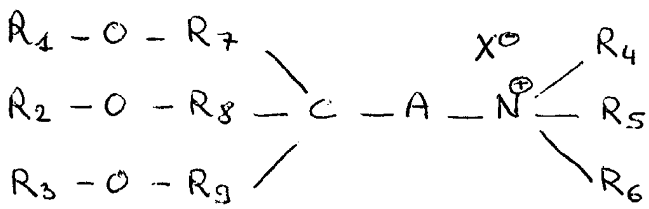

dans laquelle :

- A représente une simple liaison, un groupement NH-R', ou un groupement

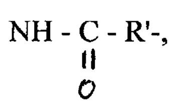 , où -R'-

est une chaîne aliphatique comportant de 1 à 22 atomes de carbone, linéaire ou ramifiée, éventuellement substituée, saturée ou insaturée, éventuellement interrompue par un ou plusieurs des hétéroatomes O, S, N, ainsi que par un ou plusieurs radicaux carbocycliques ou hétéroycliques, saturés, insaturés ou aromatiques,

- $R_1$, $R_2$, $R_3$ sont identiques ou différents et représentent chacun un groupement alkyle ou acyle supérieur,
- $R_7$, $R_8$, $R_9$ sont identiques ou différents et représentent chacun un radical alkylène $(CH_2)_n$ où $1 \leq n \leq 6$,
- $R_4$,$R_5$,$R_6$ sont identiques ou différents et représentent chacun :
  - un atome d'hydrogène
  - un radical alkyle, alcényle, alcynyle ou acyle comportant de 1 à 22 atomes de carbone, éventuellement

EP 1 245 249 A2

substitué, éventuellement interrompu par un ou plusieurs hétéroatomes choisis parmi O,S,N, ou par un ou plusieurs radicaux carbocycliques ou hétérocycliques, saturés, insaturés ou aromatiques,

- ou encore, au moins 2 des groupements R4, R5 et R6 forment ensemble avec l'atome d'azote auquel ils sont liés un groupement quinuclidino, piperidino, pyrrolidino ou morpholino,
- X est un anion non toxique,

pour son utilisation comme médicament, comme agent de transfection, ou comme adjuvant dans une composition vaccinale.

## Description

L'invention est relative au domaine des composés amphipathiques cationiques et à leur utilisation notamment comme agent de transfection ou comme adjuvant vaccinal.

La transfection, c'est-à-dire l'introduction sans dommage dans une cellule eucaryote vivante d'ADN ou d'ARNm (ARN messager) susceptible de s'exprimer, constitue une approche alternative à l'introduction intracellulaire de polypeptides et de protéines. Cette technique trouve application dans de nombreux domaines allant de la biologie cellulaire à la thérapeutique. En biologie cellulaire, les techniques de transfection peuvent être utilisées notamment pour l'étude du rôle intracellulaire des produits de gênes clonés et pour l'étude de la régulation de l'expression de gênes. La transfection est également utilisée en thérapie génique pour la correction de désordres génétiques ; elle trouve également application dans le domaine des peptides thérapeutiques et en immunisation avec le développement de vaccins polynucléotidiques.

On connaît dans l'art antérieur de nombreuses méthodes de transfection, dont notamment la précipitation d'ADN et de phosphate de calcium ou de dextran DEAE, ou encore l'utilisation de lipides cationiques sous forme de liposomes, qui forment des complexes avec les polynucléotides chargés négativement et facilitent leur passage transmembranaire. Parmi les lipides cationiques connus, on peut citer :

- le DOTMA (ou chlorure de N-[1-(2,3-dioleyloxy)propyl]-N,N,N-triméthylammonium) commercialisé en association avec un lipide neutre, la DOPE (ou dioleoylphosphatidylethanolamine) sous forme de liposomes, par la Société GIBCO BRL sous la dénomination Lipofectin™,

- des lipospermines, telles que la DOGS (5-carboxyspermylglycine-dioctadécylamide) fournie par la Société SEPRACOR sous la dénomination Transfectam™, et le DOSPA (trifluoroacétate de 2,3 dioleyloxy-N-[2(sperminecarboxamido)éthyl]-N,N-diméthyl-propanammonium) fournie par la Société GIBCO BRL sous la dénomination Lipofectamine ™,.

- des lipopolylysines (Cf. Zhou et al, Biochim. Biophys. Acta, 1065, *8-14,* 1991),

- des détergents à ammonium quaternaire, tels que le cétyltriméthylammonium ou le DDAB (bromure de diméthyldioctadécyl-ammonium) commercialisé en association avec un lipide neutre sous forme de liposomes par la Société GIBCO BRL sous la dénomination Transfectace™,

- des dérivés cationiques de cholestérol tel que le DC chol (3β[N-(N',N'-diméthylaminoéthane)-carbamoyl]cholestérol (Cf. X. Gao and L. Huang, Biochem. Biophys. Res. Commun., 1979, *280-285,* 1991),

- des analogues métabolisables de DOTMA tels que le DOTAP(1,2-dioleoyloxy-3-(triméthylammonio)propane) fourni par la Société BOEHRINGER MANNHEIM.

Bien qu'un certain nombre de ces produits soient disponibles commercialement et efficaces pour la transfection de cellules en culture, leur efficacité in vivo est limitée. Il existe donc un besoin de nouveaux agents de transfection capables de favoriser le passage de polynucléotides à travers la membrane cellulaire chargée négativement, qui soient peu ou pas toxiques et efficaces in vivo.

L'invention a pour but de proposer de tels agents de transfection, possédant des propriétés fusogènes avec la membrane de la cellule ou la membrane de l'endosome.

Pour atteindre ce but, l'invention a pour objet un composé amphipathique cationique de formule I :

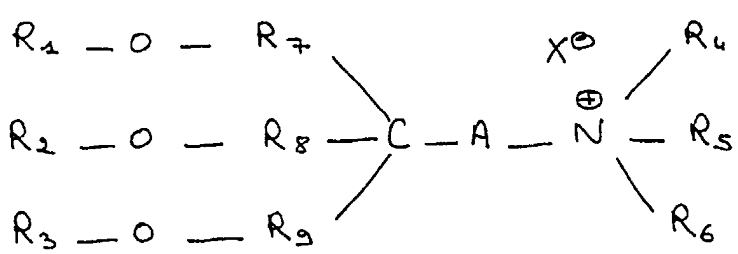

dans laquelle :

- A représente une simple liaison, un groupement NH-R', ou un groupement

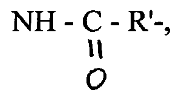

où -R'- est une chaîne aliphatique comportant de 1 à 22 atomes de carbone, linéaire ou ramifiée, éventuellement substituée, saturée ou insaturée, éventuellement interrompue par un ou plusieurs des hétéroatomes O, S, N, ainsi que par un ou plusieurs radicaux carbocycliques ou hétéroycliques, saturés, insaturés ou aromatiques,

- $R_1$, $R_2$, $R_3$ sont identiques ou différents et représentent chacun un groupement alkyle ou acyle supérieur,

- $R_7$, $R_8$, $R_9$ sont identiques ou différents et représentent chacun un radical alkylène $(CH_2)_n$ où $1 \leq n \leq 6$,

- $R_4$,$R_5$,$R_6$ sont identiques ou différents et représentent chacun :

  - un atome d'hydrogène
  - un radical alkyle, alcényle, alcynyle ou acyle comportant de 1 à 22 atomes de carbone, éventuellement substitué, éventuellement interrompu par un ou plusieurs hétéroatomes choisis parmi O,S,N, ou par un ou plusieurs radicaux carbocycliques ou hétérocycliques, saturés, insaturés ou aromatiques,
  - ou encore, au moins 2 des groupements R4, R5 et R6 forment ensemble avec l'atome d'azote auquel ils sont liés un groupement quinuclidino, piperidino, pyrrolidino ou morpholino,
  - X est un anion non toxique,

pour son utilisation comme médicament.

Selon une caractéristique particulière de l'invention $R_7$, $R_8$ et $R_9$ sont tous identiques et représentent le groupement méthylène $-CH_2-$.

Selon une autre caractéristique de l'invention $R_4$, $R_5$ et $R_6$ sont tous identiques et représentent le groupement méthyle $CH_3$-.

Selon une autre caractéristique, $R_1$, $R_2$ et $R_3$ sont tous identiques et représentent un groupement acyle supérieur, tel que le dodécanoyle ou l'hexadécanoyle.

Selon encore une autre caractéristique, A représente un groupement

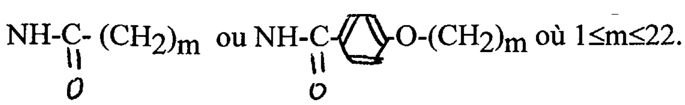

Selon un mode particulier de l'invention, le composé amphipathique cationique de formule I est associé à un lipide neutre, tel que la dioleoylphosphatidylethanolamine, dans un rapport molaire compris entre 9/1 et 1/9.

L'invention a également pour objet le composé de formule I pour son utilisation comme agent de transfection.

La présente invention a également pour objet une méthode de transfection de cellules eucaryotes, caractérisée en ce qu'elle consiste à mélanger le composé de formule I avec au moins un plasmide ou un polynucléotide, et à porter le mélange ainsi obtenu au contact des cellules à transfecter.

L'invention a également pour objet un agent de transfection caractérisé en ce qu'il comporte au moins un composé de formule I.

Selon un mode particulier de réalisation, l'agent de transfection se présente sous forme de liposomes pouvant être en suspension aqueuse ou lyophilisés.

L'invention a également pour objet une composition pharmaceutique caractérisée en ce qu'elle comprend au moins un composé de formule I.

L'invention a en outre pour objet le composé de formule I pour son utilisation comme adjuvant dans une composition vaccinale.

L'invention a aussi pour objet une composition vaccinale caractérisée en ce qu'elle comprend au moins un composé de formule I.

L'invention a encore pour objet l'utilisation d'un composé amphipathique cationique de formule I pour la fabrication d'un médicament utilisable en thérapie génique.

L'invention a de plus pour objet l'utilisation d'un composé amphipathique cationique de formule I pour la fabrication d'un médicament utilisable en vaccination.

La présente invention sera mieux comprise à la lecture de la description et des exemples qui suivent en référence aux figures qui illustrent les résultats d'un certain nombre d'essais effectués.

La figure 1 illustre les résultats obtenus à l'exemple 4, c'est-à-dire l'activité de la luciférase en pg/mg de protéines cellulaires pour l'agent de transfection obtenu selon l'exemple 1, l'agent de transfection obtenu selon l'exemple 2 et un agent de transfection de l'art antérieur.

La figure 2 illustre les résultats obtenus à l'exemple 5.

La figure 3 illustre les résultats obtenus à l'exemple 6 , dans les poumons des souris.

La figure 4 illustre les résultats obtenus à l'exemple 6, dans les glandes salivaires des souris.

La figure 5 illustre les résultats obtenus à l'exemple 7, dans les poumons et la trachée des souris.

Aux termes de la présente invention , on entend par polynucléotide toute molécule constituée par un enchaînement de nucléotides, telle que ADN, ARN ou triple hélice.

Par radical alkyle, on entend tout radical hydrocarboné possédant de préférence de 1 à 32 atomes de carbone et comportant uniquement des liaisons simples. Par radical alkyle inférieur, on entend un radical comprenant de 1 à 10 atomes de carbone ; il peut notamment s'agir du radical méthyle, éthyle, propyle, isopropyle, butyle, isobutyle, n-pentyle, n-hexyle, 2-méthylpentyle, 2,2-dimethyl-pentyle, 3,3-diméthyl pentyle, 3 éthylpentyle, n-octyle, 2,2-diméthylhexyle, 3,3-diméthylhexyle, 3-méthyl 3-éthyl pentyle, n-nonyle, n-décyle. Par radical alkyle supérieur, on entend un radical comprenant plus de 10 atomes de carbone ; il peut notamment s'agir des radicaux undecyle, dodecyle, tridecyle, tétradecyle, pentadecyle, hexadecyle, heptadecyle, octadecyle, nonadecyle, eicosyle, hénéicosyle, docosyle, triacontyle.

Par radical alcényle, on entend tout radical hydrocarboné comportant au moins une double liaison mais pas de triple liaison. Les radicaux alcényles selon l'invention possèdent de préférence de 2 à 32 atomes de carbone. Par radical alcényle inférieur, on entend un radical ayant de 2 à 10 atomes de carbone tel que le radical vinyle, allyle, propenyle, isopropenyle, 2-methyl allyle, butényle ou isobuténylе. Par radical alcényle supérieur, on entend un radical ayant plus de 10 atomes de carbone.

Par radical alcynyle, on entend tout radical hydrocarboné comportant au moins une triple liaison. Les radicaux alcynyle selon l'invention contiennent de préférence de 2 à 32 atomes de carbone. Par radical alcynyle inférieur, on entend un radical ayant de 2 à 10 atomes de carbone, tel que le radical éthynyle, propynyle, propargyle, butynyle ou isobutynyle. Par radical alcynyle supérieur, on entend un radical ayant plus de 10 atomes de carbone.

Par radical acyle, on entend un radical

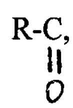

où R est un radical alkyle, alcényle ou alcynyle tel que défini ci-dessus. On entend par radical acyle inférieur un radical

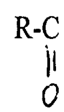

dans lequel R contient au plus 10 atomes de carbone, et par radical acyle supérieur un radical dans lequel R contient plus de 10 atomes de carbone, tels que les radicaux acyles dérivés des acides suivants : acide laurique, myristique, palmitique, stéarique, arachidique, béhénique, lignocérique, cérotique.

On préfère utiliser les radicaux suivants : dodécanoyle et hexadecanoyle dérivés respectivement de l'acide laurique et de l'acide palmitique.

Par radical carbocyclique saturé, on entend par exemple le radical cyclopropyle, cyclobutyle, cyclopentyle, ou cyclohexyle.

Par radical carbocyclique insaturé, on entend par exemple le radical cyclobuténylе, cyclopenténylе, cyclo-

pentadiényle, cyclohexényle ou cyclohexadiényle.

Par radical hétérocyclique saturé, on entend par exemple le radical pyrrolidinyle, pipéridinyle, imidazolidinyle, pyrazolidinyle, piperazinyle, morpholinyle, thiomorpholinyle, ou azépinyle.

Par radical aromatique, carbocyclique ou hétérocyclique, on entend par exemple le radical phényle, thiényle, furyle, pyrranyle, pyrrolyle, imidazolyle, pyrazolyle, pyridyle, pyrazinyle, pyrimidinyle, pyridazinyle, thiazolyle, oxazolyle, furazannyle, pyrrolinyle, imidazolinyle, pyrazolinyle, thiazolinyle, triazolyle, tétrazolyle, cyclopropanyle, cyclobutanyle, cyclopentanyle, cyclohexanyle, cycloheptanyle, cyclooctanyle... etc.

L'expression "éventuellement substitué(e)" appliquée à la chaîne aliphatique R' et aux radicaux alkyle, alcényle, alcynyle ou acyle que peuvent représenter $R_4$, $R_5$ et $R_6$ indique que ceux-ci peuvent être éventuellement substitués par un ou plusieurs radicaux identiques ou différents choisis parmi notamment les radicaux suivants :

- halogène : fluor, chlore, brome, iode,
- amino, alkylamino tel que méthylamino ou éthylamino, dialkylamino tel que diméthylamino, diéthylamino, méthyléthylamino,
- hydroxyle éventuellement acylé, par exemple acétoxy,
- carboxy estérifié tel que alkoxy carbonyle par exemple méthoxy carbonyle ou éthoxycarbonyle,
- oxo, cyano, nitro, formyle,
- acyle tel que acétyle, propionyle, butyryle, benzoyle,
- acyloxy tel que acétoxy,
- alkoxy tel que méthoxy, éthoxy, propyloxy, isopropyloxy, butyloxy,
- alkylthio tel que méthylthio, éthylthio, propylthio, isopropylthio, butylthio,
- carbamoyle,

Les composés amphiphatiques selon l'invention sont des sels dont l'anion doit être un anion pharmaceutiquement non-toxique provenant d'acides organiques ou minéraux. Parmi ces acides, on peut citer notamment l'acide chlorhydrique, l'acide bromhydrique, l'acide sulfurique, l'acide phosphorique, l'acide acétique, l'acide benzoïque, l'acide citrique, l'acide glutamique, l'acide lactique. Pour la préparation de sels pharmaceutiquement acceptables, on peut se référer à la publication de Berge S.M. et al. J. Pharm. Sci.,66:*1-19* (1977). De façon particulière, on utilise des sels de l'acide bromhydrique.

Les composés amphipathiques cationiques convenant aux fins de l'invention peuvent être obtenus par toute méthode classique d'acylation ou d'alkylation d'aminoalcools ; on peut, par exemple, utiliser les méthodes décrites dans "Advanced Organic Chemistry" Part B : Reactions and Synthesis (F.A. Carey and R.J.Sundberg - Plenum Publishing Corp.). Le composé aminoalcool de départ peut être de l'hydroxyméthylhydroxyethylhydroxypropylaminométhane, du di(hydroxyméthyl) hydroxyéthylaminométhane, du di(hydroxyéthyl)hydroxyméthylaminométhane, du tris-(hydroxyméthyl)aminométhane ou un homologue supérieur tel que le tris (hydroxyéthyl)aminométhane, le tris(hydroxypropyl)aminométhane, le tris (hydroxybutyl) aminométhane, le tris(hydroxypentyl)aminométhane ou encore le tris(hydroxyhexyl) aminométhane. Avantageusement, on utilise comme composé de départ du tris(hydroxyméthyl)aminométhane.

Le groupement constituant la tête cationique du composé amphipathique selon l'invention peut être généré par alkylation directe du groupement aminé du composé aminoalcool de départ(dans ce cas, A, indiqué dans la formule générale mentionnée ci-dessus, représente la liaison simple existant entre l'atome de carbone C et l'atome d'azote N). De façon préférentielle, la tête cationique est reliée au groupement azoté via un bras espaceur A, par alkylation ou acylation réalisée au moyen d'un réactif alkylant (halogénure d'alkyle) ou acylant (acide carboxylique activé) chargé positivement ou pouvant être modifié de manière à lui conférer une charge positive.

La partie lipophile des composés amphipathiques convenant à la présente invention est constituée par 3 chaînes hydrocarbonées $R_1$, $R_2$, $R_3$ identiques ou différentes. Ces 3 chaînes proviennent soit d'alcool gras activés, soit d'acides gras activés qui ont réagi avec la fonction alcool du composé aminoalcool de départ pour former respectivement une liaison éther ou ester. De façon particulière, on utilise des composés obtenus à partir de l'acide dodécanoïque, ou à partir de l'acide hexadécanoïque.

Parmi les composés convenant aux fins de l'invention, on peut citer notamment les composés dont la formule est la suivante :

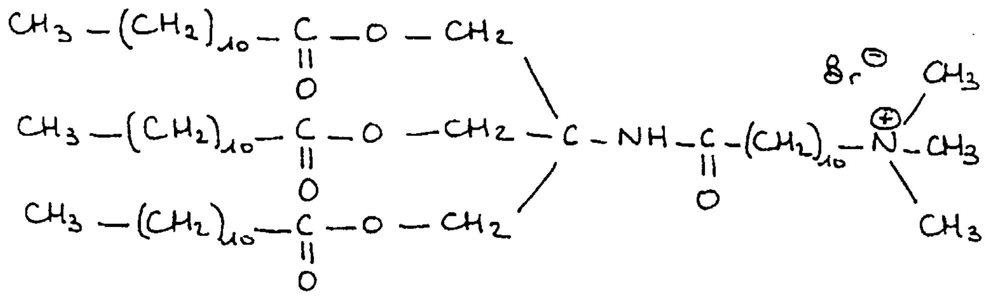

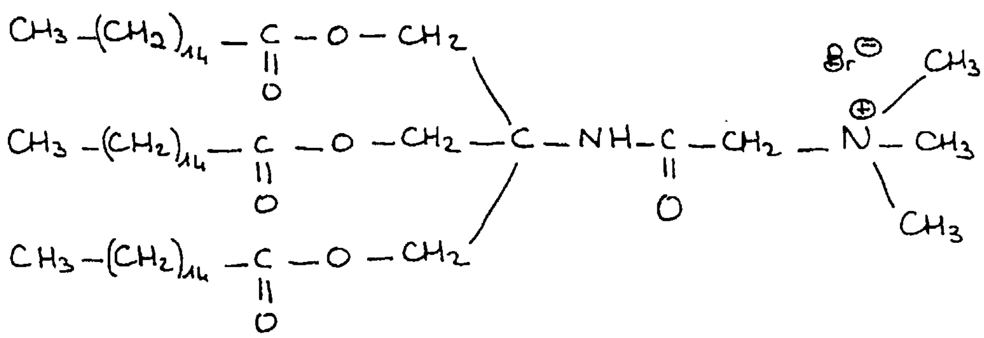

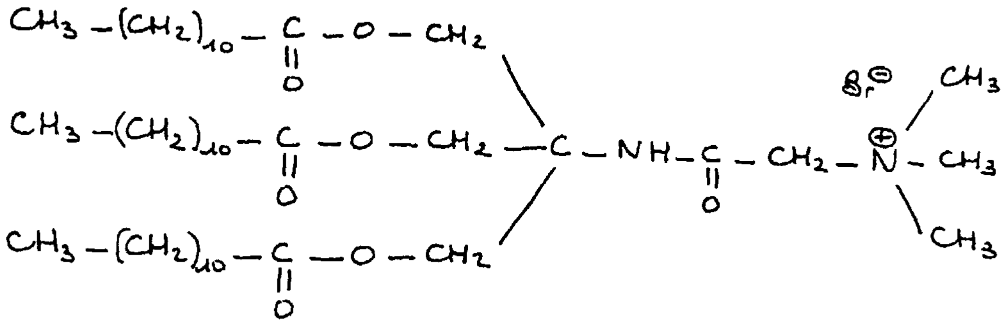

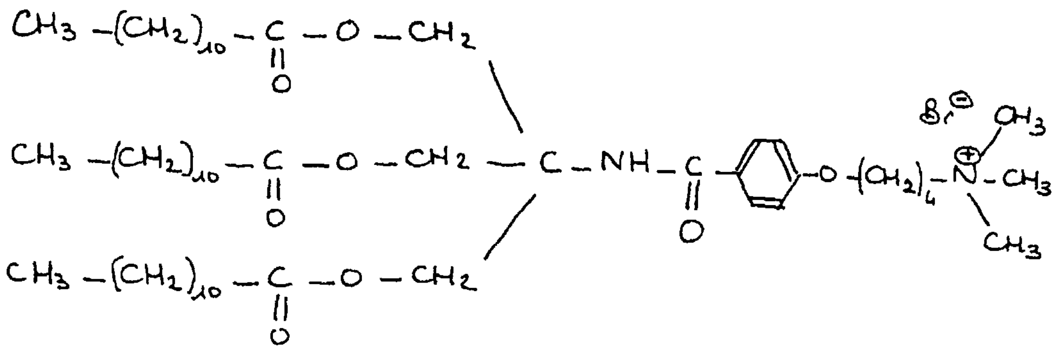

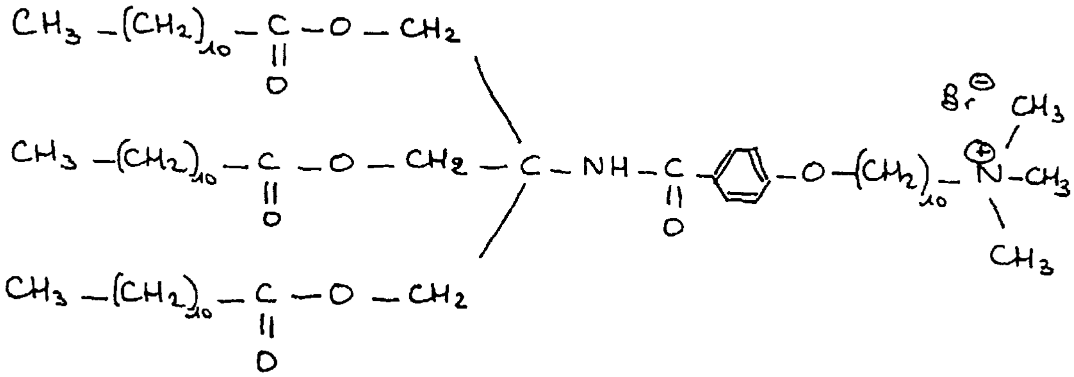

dont la méthode de fabrication est décrite dans l'article "Bilayer Membranes of Triple-Chain Ammonium Amphiphiles" Kunitake et al. J. Am. Chem. Soc.1984, 106, 1978-1983.

Selon un mode de réalisation de l'invention, le composé amphipathique cationique est associé à au moins un lipide neutre, tel que le cholestérol, une phosphatidylcholine ou une phosphatidyléthanolamine. On a obtenu de particulièrement bons résultats en associant les composés amphipathiques cationiques à de la dioléoylphosphatidyléthanolamine (DOPE). La proportion molaire de composé amphipathique cationique et de lipide neutre est avantageusement comprise entre 9/1 et 1/9. De façon particulière, on utilise un rapport 1/2.

Par la suite, pour des raisons de simplification, on parlera de formulation lipidique selon l'invention pour signifier soit le composé amphipathique cationique seul, soit le composé amphipathique cationique associé à au moins un lipide neutre, tel que la dioléoylphosphatidyléthanolamine.

Selon un mode de réalisation, la formulation lipidique de la présente invention est organisée sous forme de liposomes à paroi simple ou multiple. Ces liposomes sont obtenus lors de la dispersion en milieu aqueux de la formulation lipidique. Afin d'obtenir des liposomes de taille uniforme, on peut traiter la formulation lipidique selon l'invention par toute méthode connue à cette fin par l'homme du métier et publiée dans la littérature (ex : Liposomes : A practical Approach RRC New Ed. IRL Press, Oxford University Press, 1990). Il est notamment possible de procéder à l'injection

rapide dans un milieu aqueux d'une solution éthanolique de formulations lipidiques ou à la sonication de liposomes formés spontanément à partir des formulations lipidiques en milieu aqueux.

Selon l'invention, lorsque le composé de formule I est destiné à être utilisé comme agent de transfection, les formulations lipidiques sont associées aux polynucléotides que l'on souhaite introduire dans les cellules, pour former des complexes par interaction de charges. Cette association peut être réalisée par simple mélange des différents composés en solution.

L'agent de transfection selon l'invention permet de transfecter de nombreuses cellules ; on peut par exemple transfecter des lignées de cellules adhérentes (ex : CHO - K1, A549), des cellules primaires (ex : MRC-5) et des lignées de cellules en suspension (ex : K562).

On a pu, avec les cellules CHO, atteindre des taux de transfection allant jusqu'à 50 pour cent.

Grâce à l'agent de transfection selon l'invention, on peut transfecter dans un volume final de 100 μl des quantités élevées d'ADN plasmidique (jusqu'à 60 μg) ; ce résultat est important pour une application en thérapie génique où il est nécessaire de délivrer une grande quantité de polynucléotides dans un volume compatible avec une administration parentérale ou mucosale.

Le composé selon l'invention peut également être utilisé comme adjuvant dans des compositions vaccinales afin de modifier la réponse immunitaire. Dans ce cas, il est possible d'obtenir une composition vaccinale par simple mélange d'antigènes vaccinaux et de liposomes ou par encapsulation des Ag par des liposomes, les liposomes étant obtenus à partir de formulation lipidique selon l'invention ainsi que cela vient d'être décrit.

L'utilisation du composé selon l'invention comme agent de transfection ou comme adjuvant vaccinal n'a entraîné aucun signe de toxicité. En effet, l'observation au microscope de cellules transfectées n'a révélé aucun signe de cyto-toxicité. En outre, lors du transfert in vivo de gènes dans la trachée et les poumons de souris, ainsi que lors des essais d'immunisation de souris par la voie sous-cutanée, aucun signe de toxicité aigüe n'est apparu.

Les exemples suivants illustrent, de façon non limitative, quelques modes de mise en oeuvre de l'invention.

<u>Exemple 1</u>

On dispose de bromure de O,O',O"-tridodécanoyl-N-(ω triméthylammoniododécanoyl)-tris-(hydroxyméthyl) aminométhane fourni par SOGO sous la dénomination TC-1-12. On en dissoud 21 mg dans 75 μl d'éthanol. 50 μl de cette solution éthanolique (soit 15 μmol) sont ensuite injectés rapidement à l'aide d'une seringue Hamilton dans 2 ml d'eau désionisée sous agitation à 45°C. On obtient dans ces conditions des petites vésicules unilamellaires de composé amphipathique cationique dont le diamètre moyen est de 50 nm.

<u>Exemple 2</u>

On mélange, dans du chloroforme 1,4 mg (soit 1,5 μmol) de bromure de O,O',O"- tridodécanoyl-N-(ω triméthylammoniododécanoyl)-tris-(hydroxyméthyl) aminométhane et 2,24 mg (soit 3 μmol) de dioleoylphosphatidyléthanolamine (DOPE) que l'on fait sécher sur les parois d'un récipient en verre dans un évaporateur rotatif ; le mélange de lipides est remis en suspension dans 2 ml d'eau désionisée stérile. Après réhydratation pendant une nuit à 4°C, la dispersion est soumise à sonication pendant 10 min pour former de petits liposomes unilamellaires, dont le diamètre moyen est de 100 à 200 nm.

<u>Exemple 3</u>

Les suspensions de liposomes obtenues selon l'exemple 1 ou l'exemple 2 sont diluées dans de l'eau désionisée pour atteindre une concentration de 0,747 mM de charges positives et sont utilisées pour transfecter des cellules CHO en culture selon la méthode décrite par Felgner J. et al. J. Tiss. Cult. Meth., <u>15</u>:*63-68*, 1993.

Les cellules CHO sont mises en culture dans une plaque à 96 puits (10 à 30 000 cellules par puits) un jour avant l'essai de transfection.

On prépare des complexes liposomes-ADN possédant différentes quantités d'ADN et de liposomes de la façon suivante :

1) Dilution de la formulation lipidique

On distribue 66 μl de milieu αMEM dans chaque puits de la 1ère colonne d'une plaque à 96 puits (c'est-à-dire les puits $A_1$ à $H_1$) et 60 μl dans tous les autres puits. On met 54 μl de la solution à 0,747 mM de charges positives de la formulation lipidique obtenue selon l'exemple 1 ou l'exemple 2 dans chaque puits de la 1ère colonne. On effectue ensuite une dilution en série de la solution lipidique en transférant 60 μl de chaque puits de la colonne 1 au puits correspondant de la colonne 2, puis 60 μl de chaque puits de la colonne 2 au puits correspondant de la colonne 3, et ainsi de suite jusqu'à la colonne 8 (puits $A_8$- $H_8$).

2) Dilution du plasmide

Le plasmide avec lequel on souhaite transfecter des cellules est le plasmide pCMV-β Gal qui, lorsqu'il y a transfection, va conduire la cellule à produire une enzyme, la β Galactosidase, dont il est possible de mesurer faoilement l'activité. On réalise différentes dilutions de la solution plasmidique grâce encore à une plaque à 96 puits contenant chacun 70 µl de milieu αMEM. On met 70 µl d'une solution plasmidique contenant 80 µg de pCMV-βGal/ml de milieu αMEM dans chaque puits de la première rangée (puits $A_1$-$A_8$). La solution plasmidique est, ensuite, diluée en série en transférant 70 µl de la solution plasmidique de chaque puits de la première rangée dans le puits correspondant de la seconde rangée, puis 70 µl de la solution de chaque puits de la seconde rangée dans le puits correspondant de la troisième rangée et ainsi de suite jusqu'à la huitième rangée (puits $H_1$ - $H_8$).

Les complexes liposomes/ADN correspondant à différentes quantités d'ADN et à différentes valeurs de la charge des liposomes sont obtenus en transférant 60 µl de chaque puits de la plaque contenant les plasmides dans le puits correspondant de la plaque contenant les liposomes. Après 10 minutes d'incubation à température ambiante, 100 µl des complexes résultants sont transférés sur les couches de cellules. Les cellules sont incubées à 37°C en atmosphère humide comportant 5% de $CO_2$. On ajoute aux cultures du sérum 5 heures après la transfection (par addition de 50 µl deαMEM contenant 30 % de sérum de veau foetal) ainsi que 24 h après la transfection (addition cette fois de 100 µl de αMEM contenant 10 % de sérum de veau foetal).

L'efficacité de la transfection est mesurée à 48 heures après lyse des cellules, en utilisant de l'ONPG (ortho nitrophényl galactoside) comme substrat pour détecter et mesurer selon une méthode colorimétrique l'activité de la βgalactosidase fabriquée par les cellules. La βgalactosidase transforme l'ONPG incolore en galactose et orthonitrophénol coloré en jaune (absorption à 405 nm).

Les résultats obtenus sont représentés sur les tableaux ci-après où on indique, pour chaque puits, la densité optique lue à 405 nm.

Pour chaque tableau, les puits de la 9ème colonne correspondent à des témoins négatifs représentant le bruit de fond, alors que les puits de la 10ème colonne sont utilisés pour faire une gamme étalon de la βgalactosidase ; le 1er puits de la dixième colonne correspond à une concentration de 400 ng/ml, le 2ème à 200 ng/ml, le 3ème à 100 ng/ml, et ainsi de suite à des concentrations diminuées à chaque fois de moitié, jusqu'au 8ème puits où la concentration est de 3,125 ng/ml.

Le tableau 1 indique les résultats obtenus avec les liposomes de l'exemple 1.

TABLEAU 1

| | 1 | 2 | 3 | 4 | 5 | 6 | 7 | 8 | 9 | 10 |
|---|---|---|---|---|---|---|---|---|---|---|
| A<br>2 µg ADN | 2.456 | 3.317 | 3.446 | 3.300 | 1.265 | 0.396 | 0.230 | 0.152 | 0.131 | 3.259 |
| B<br>1 µg ADN | 0.876 | 3.281 | 3.289 | 3.551 | 1.417 | 0.362 | 0.190 | 0.145 | 0.146 | 3.299 |
| C<br>0,5 µg ADN | 0.255 | 1.027 | 3.581 | 3.438 | 2.331 | 0.583 | 0.315 | 0.141 | 0.127 | 2.383 |
| D<br>0,25 µg ADN | 0.185 | 0.404 | 3.247 | 3.050 | 2.586 | 0.639 | 0.216 | 0.137 | 0.129 | 1.289 |
| E<br>0,125 µg ADN | 0.167 | 0.256 | 1.736 | 2.145 | 2.422 | 0.490 | 0.169 | 0.139 | 0.135 | 0.745 |
| F<br>0,062 µg ADN | 0.174 | 0.248 | 0.829 | 0.799 | 0.691 | 0.296 | 0.151 | 0.132 | 0.131 | 0.472 |
| G<br>0,031 µg ADN | 0.173 | 0.184 | 0.536 | 0.319 | 0.388 | 0.333 | 0.146 | 0.140 | 0.140 | 0.364 |
| H<br>0,015 µg ADN | 0.169 | 0.185 | 0.377 | 0.270 | 0.201 | 0.210 | 0.216 | 0.144 | 0.155 | 0.257 |
| nbre de nmoles de charges + | 17 | 8,5 | 4,25 | 2,13 | 1,06 | 0,53 | 0,27 | 0,13 | | |

Le tableau 2 indique les résultats obtenus avec les liposomes de l'exemple 2.

TABLEAU 2

| | 1 | 2 | 3 | 4 | 5 | 6 | 7 | 8 | 9 | 10 |
|---|---|---|---|---|---|---|---|---|---|---|
| A<br>2 µg ADN | 1.205 | 1.819 | 2.821 | 2.452 | 2.349 | 2.322 | 1.688 | 1.115 | 0.121 | 3.347 |
| B<br>1 µg ADN | 0.467 | 1.225 | 2.460 | 2.448 | 2.362 | 2.479 | 2.302 | 1.377 | 0.118 | 3.283 |
| C<br>0,5 µg ADN | 0.459 | 1.004 | 2.090 | 2.547 | 2.175 | 2.307 | 2.187 | 1.599 | 0.116 | 2.194 |
| D<br>0,25 µg ADN | 0.363 | 0.958 | 1.892 | 2.001 | 2.467 | 2.397 | 2.405 | 1.659 | 0.119 | 1.168 |
| E<br>0,125 µg ADN | 0.273 | 0.795 | 1.592 | 2.318 | 2.267 | 2.405 | 2.085 | 1.488 | 0.122 | 0.667 |
| F<br>0,062 µg ADN | 0.275 | 0.578 | 1.188 | 1.746 | 2.084 | 2.435 | 1.905 | 1.403 | 0.121 | 0.430 |
| G<br>0,031 µg ADN | 0.175 | 0.402 | 0.915 | 1.106 | 1.682 | 1.915 | 1.330 | 0.911 | 0.124 | 0.336 |
| H<br>0,015 µg ADN | 0.166 | 0.233 | 0.556 | 0.585 | 0.892 | 1.209 | 1.040 | 0.546 | 0.139 | 0.236 |
| nbre de nmoles de charges + | 17 | 8,5 | 4,25 | 2,13 | 1,06 | 0,53 | 0,27 | 0,13 | | |

Le tableau 3 indique les résultats obtenus avec un agent de transfection de l'art antérieur : le DOTMA commercialisé sous la dénomination Lipofectin ™.

TABLEAU 3

| | 1 | 2 | 3 | 4 | 5 | 6 | 7 | 8 | 9 | 10 |
|---|---|---|---|---|---|---|---|---|---|---|
| A<br>2 µg ADN | 0.322 | 0.747 | 3.189 | 3.124 | 3.283 | 2.619 | 1.883 | 1.350 | 0.133 | 3.289 |
| B<br>1 µg ADN | 0.234 | 0.334 | 1594 | 3.454 | 3.256 | 3.141 | 2.362 | 1.423 | 0.138 | 3.249 |
| C<br>0,5 µg ADN | 0.200 | 0.205 | 1.060 | 2.977 | 3.549 | 3.043 | 2.288 | 1.099 | 0.130 | 2.585 |
| D<br>0,25 µg ADN | 0.190 | 0.170 | 0.666 | 1.802 | 3.099 | 3.397 | 1.866 | 1.073 | 0.129 | 1.506 |
| E<br>0,125 µg ADN | 0.173 | 0.143 | 0.289 | 0.758 | 2.022 | 3.443 | 1.635 | 0.619 | 0.125 | 0.855 |
| F<br>0,062 µg ADN | 0.170 | 0.139 | 0.158 | 0.371 | 0.733 | 1.449 | 1.595 | 0.585 | 0.129 | 0.565 |
| G<br>0,031 µg ADN | 0.167 | 0.138 | 0.136 | 0.223 | 0.334 | 0.513 | 0.615 | 0.294 | 0.131 | 0.392 |
| H<br>0,015 µg ADN | 0.169 | 0.135 | 0.139 | 0.171 | 0.216 | 0.302 | 0.318 | 0.254 | 0.141 | 0.334 |
| nbre de nmoles de charges + | 17 | 8,5 | 4,25 | 2,13 | 1,06 | 0,53 | 0,27 | 0,13 | | |

Les résultats obtenus montrent l'efficacité de la transfection effectuée, soit avec des liposomes constitués uniquement du composé amphipathique cationique selon l'invention, soit avec des liposomes constitués à la fois du composé amphipathique cationique selon l'invention et d'un colipide neutre.

Exemple 4

On évalue l'efficacité de la transfection réalisée avec des formulations lipidiques selon l'invention et avec un agent de l'art antérieur, sur différents types de cellules : les cellules A 549 (lignées de cellules épithéliales de carcinome de poumon humain), les cellules MRC5 (fibroblastes pulmonaires foetaux humains) et sur une lignée de cellules en suspension, les cellules K 562 (lymphoblastes dérivés d'une leucémie chronique humaine myélogène).

Les cellules sont transfectées cette fois par le plasmide pCMV-Luc qui, lorsqu'il s'exprime, conduit à la fabrication par les cellules d'une enzyme : la luciférase, dont on peut doser l'activité en utilisant comme substrat la luciférine, selon la technique décrite dans l'article "Firefly Luciferase Gène : Structure and Expression in Mammalian Cells." (J. R. De Wet et al, Mol. Cell Biol., 7, 725-737, 1987).

Les cellules adhérentes sont semées un jour avant l'essai de transfection dans des boîtes de Pétri de 60 mm à une densité leur permettant d'atteindre une confluence de 1/2 - 3/4 au moment de la transfection. On prépare les complexes plasmide/agent de transfection en ajoutant 5 μg de plasmide en suspension dans un milieu de culture approprié contenant du sérum (milieu αMEM pour cellules MRC5, milieu DMEM pour cellules A 549, et milieu RPMI 1640 pour cellules K 562) à 0,5 ml du même milieu contenant une quantité de liposomes permettant d'obtenir un rapport charges +/charges - de 0,7 pour la Lipofectin™ ainsi que pour l'agent de transfection obtenu selon l'exemple 2, et un rapport égal à 1,4 pour l'agent de transfection obtenu selon l'exemple 1. Après mélange et incubation pendant 10 minutes à température ambiante, on ajoute les complexes résultants aux cellules et on met à incuber à 37°C en atmosphère humide contenant 5 % de $CO_2$.

On ajoute du sérum aux cultures 5 heures après la transfection (par addition de 0,5 ml du milieu approprié contenant 30 % de sérum de veau foetal) ainsi que 24 heures après la transfection (par addition cette fois d'1 ml du milieu approprié contenant 10 % de sérum de veau foetal). L'efficacité de la transfection est appréciée à 48 heures, en utilisant de la luciférine comme substrat selon la technique mentionnée ci-dessus.

Pour transfecter des cellules en suspension, on prépare des complexes plasmide pCMV-Luc/formulations lipidiques à partir de milieu RPMI 1640 ainsi que cela est décrit ci-dessus. On utilise ensuite la solution obtenue pour remettre en suspension $1,5.10^6$ cellules K 562 dans des boîtes de Petri de 35 mm. On ajoute du sérum dilué et on apprécie la transfection ainsi que cela a été décrit ci-dessus pour les cellules adhérentes.

Les résultats obtenus sont représentés sur l'histogramme de la figure 1, où on peut voir que tous les types de cellules ont pu être transfectés.

Exemple 5

On réalise la transfection de cellules CHO avec des complexes concentrés ADN/formulations lipidiques.

On prépare des complexes en ajoutant une suspension de formulation lipidique dans 50 μl d'eau désionisée à une solution contenant 60 μg de plasmide pCMV-Luc dans 50 μl de sérum physiologique. La quantité de lipides est ajustée pour obtenir des rapports charges +/charges- de 0.35, 0.7 ou 1.4 dans les complexes résultants. Après mélange et incubation à température ambiante pendant 10 minutes, on ajoute les complexes résultants à 2 ml de milieu optiMEM (fourni par la Société GIBCO BRL) et on étend le mélange obtenu sur des couches de cellules confluentes au 3/4. Les cellules sont ensuite mises à incuber avec les complexes pendant 4 heures à 37°C en atmosphère humide contenant 5 pour cent de $CO_2$. On remplace ensuite le milieu par 3 ml de α MEM contenant 10 pour cent de sérum de veau foetal. On mesure l'efficacité de la transfection après 48 heures, en utilisant de la luciférine ainsi que cela a été décrit ci-dessus. Les résultats obtenus sont représentés sur l'histogramme de la figure 2, et montrent qu'il est possible de transfecter des cellules, même en utilisant de fortes concentrations d'ADN. On n'a pas observé de différence significative dans les résultats lorsqu'au lieu d'ajouter les liposomes à la solution d'ADN, on ajoutait la solution d'ADN à la solution contenant la formulation lipidique selon l'invention.

Exemple 6

On effectue un transfert de gène in-vivo en vue d'induire des réponses immunitaires locales. On utilise 30 μg de plasmide pCMV-HA codant pour l'hémagglutinine du virus de la grippe (Souche APR8) dans 25 μg d'eau physiologique (solution aqueuse de NaCl à 9 g/l) que l'on ajoute à 25 μl d'eau désionisée contenant ou non des liposomes cationiques. Une des formulations ne contient que du plasmide et de l'eau désionisée et sera utilisée pour un essai de transfert d'ADN nu ; une formulation contient des liposomes tels que ceux obtenus à l'exemple 1 ; une formulation contient des liposomes tels que ceux obtenus à l'exemple 2 ; la dernière formulation contient des liposomes obtenus par mélange de 3β[N-(N',N'-diméthylaminoethane)-carbamoyl]cholestérol (DC chol) et de dioleoylphosphatidylethanolamine (DOPE) dans un rapport molaire 1/2. Pour chacune de ces formulations, la quantité de liposomes utilisés est ajustée de manière à ce que dans les complexes formés, les amines cationiques des liposomes représentent 35 % en mole des phosphates de l'ADN plasmidique. Les 50 μl de solution résultant de chaque formulation sont administrés par voie intranasale (soit 25 µl par narine) à des souris Balb/c (5 souris par groupe) anesthésiées à l'aide d'une injection intrapéritonéale de 200 µl d'un mélange ROMPUN™ (BAYER)/IMALGENE ™(Rhône -Mérieux). 21 jours après l'administration du plasmide pCMV-HA, les souris sont sacrifiées ; on prélève leurs poumons et glandes salivaires et on isole les lymphocytes à partir de ces organes. On dépose ensuite ces lymphocytes, en suspension dans du milieu MEM à 10 % de sérum de veau foetal, dans des plaques à 96 puits avec support en nitrocellulose (Multiscreen Membrane HATF, Millipore) préalablement saturé avec l'antigène étudié (HA du vaccin grippe monovalent, souche A Singapore).

Après 14 heures d'incubation dans une atmosphère à 5 % de $CO_2$ et saturée en eau, on lyse les cellules, on rince les puits, et on révèle les membranes par lavage successif avec des Anti-anticorps biotinylés (Anti-IgA et Anti-IgG de souris) puis avec une solution de streptavidine couplée à la peroxydase et enfin avec une solution de chromogène.

L'endroit où se trouvait un lymphocyte B sécréteur d'anticorps apparaît sous forme d'un spot rougeâtre qui diffuse de façon radiale dans la membrane du puits de culture. Les spots correspondant aux lymphocytes B sécréteurs des anticorps d'un isotype donné (IgG ou IgA) sont numérés sous la loupe binoculaire et leur nombre dans les poumons et dans les glandes salivaires pour chacune des 5 souris de chaque groupe est reporté sur les graphes des figures 3 et 4. Les souris 1 à 5 ont reçu la formulation d'ADN nu. Les souris 6 à 10 ont reçu la formulation comportant l'agent de transfection obtenu selon l'exemple 2 ; les souris 11 à 15 ont reçu la formulation comportant l'agent de transfection obtenu selon l'exemple 1 ; les souris 16 à 20 ont reçu la formulation comportant un agent de transfection de l'art antérieur (DC chol/DOPE).

Les résultats obtenus montrent qu'une administration unique de 30 µg d'ADN complexés au composé amphipathique de l'invention obtenu selon l'exemple 1 permet d'induire des lymphocytes B sécréteurs d'IgA et d'IgG dans les poumons et les glandes salivaires des animaux testés. Cette réponse est plus forte et plus homogène que celle obtenue respectivement avec de l'ADN nu ou avec de l'ADN complexé à des liposomes de l'art antérieur DCchol/DOPE.

Exemple 7

On réalise un transfert de gène marqueur in vivo de façon identique au transfert réalisé dans l'exemple 6, mais en partant cette fois de 30 µg de plasmide pGL3™ (Promega, France) codant pour la luciférase.Les formulations testées sont, comme à l'exemple 6, une formulation contenant des liposomes tels que ceux obtenus à l'exemple 1, une formulation contenant des liposomes tels que ceux. obtenus à l'exemple 2, une formulation de l'art antérieur contenant des liposomes DC-Chol/DOPE. Les souris testées sont sacrifiées 3 jours après l'administration des différentes formulations, les testées sont sacrifiées 3 jours après l'administration des différentes formulations, les poumons et trachées sont prélevés séparément et broyés. On dose la luciférase dans les broyats d'organe par bioluminescence. Une courbe étalon a été réalisée en mélangeant des quantités connues de luciférase purifiée à des broyats de poumons et de trachées.

On remarque que l'expression la meilleure est obtenue pour la transfection réalisée au moyen de la formulation contenant les liposomes obtenus à l'exemple 1. Ainsi, grâce à l'agent de transfection selon l'invention, il est possible d'effectuer la transfection in vivo du gène de la luciférase.

Exemple 8

On teste l'activité adjuvante d'un composé selon l'invention. A cette fin, on prépare une suspension liposomale à partir de 4 mg de bromure de 0, 0', 0"-tridodecanoyl-N-($\overline{\omega}$ trimethylammoniododecanoyl)-tris-(hydroxymethyl)aminométhane (TC-1-12 fourni par SOGO) que l'on met en solution dans 50 µl d'éthanol à 42°C et que l'on injecte rapidement à l'aide d'une seringue Hamilton dans 1770 µl d'eau maintenue sous agitation à 42°C. On maintient l'agitation pendant 2 min à 42°C puis on refroidit à température ambiante la suspension liposomale obtenue, et on ajoute ensuite goutte à goutte, à la suspension liposomale maintenue sous agitation, 230 µl de vaccin grippe monovalent NIB 16 (souche A Singapour) qui contient 220 µg d'hémagglutine HA par ml. Le mélange obtenu est ensuite divisé en 10 doses de 200 µl contenant chacune 5 µg de HA et 400 µg de composé amphipathique cationique.
On immunise 6 souris Balb/c femelles âgées de 8 à 10 semaines par injections sous-cutanées à J0 et J21 d'une dose de 200 µl de la composition préparée de la manière qui vient d'être décrite. 2 semaines après l'injection de rappel, on réalise une saignée au niveau du sinus rétro-orbital des souris. En parallèle, on administre à 4 souris utilisées comme témoin 200 µl de vaccin NIB 16 dilué à 25 µg d'hémagglutinine HA/ml à l'aide de tampon PBS.

On dose ensuite par ELISA, pour chacune des souris testées, le taux sérique en anticorps IgG1 et IgG2a contre le vaccin NIB 16.

Les résultats obtenus sont représentés sur le tableau 4 ci-après, et montrent une forte activité adjuvante de la composition liposomale selon l'invention. Le taux accru d'IgG1 et d'IgG2a montre que l'on a une action favorisant le développement à la fois des lymphocytes de type TH1 et des lymphoytes de type TH2.

TABLEAU 4

| SOURIS | IgG1 | | IgG2a | |
|---|---|---|---|---|
| | TITRE | MOYENNE GEOMETRIQUE | TITRE | MOYENNE GEOMETRIQUE |
| Composé amphipathique selon l'invention | 55 800 | 37210 | 20 900 | 16 428 |
| | 14 690 | | 5 320 | |
| | 41 580 | | 4 780 | |
| | 37 520 | | 33 990 | |
| | 50 220 | | 170 000 | |
| | 41 330 | | 16 550 | |
| Témoins | <200 | <200 | 44 | 68 |
| | <200 | | 64 | |
| | <200 | | 64 | |
| | <200 | | 118 | |

**Revendications**

**1.** Composé amphipathique cationique de formule I :

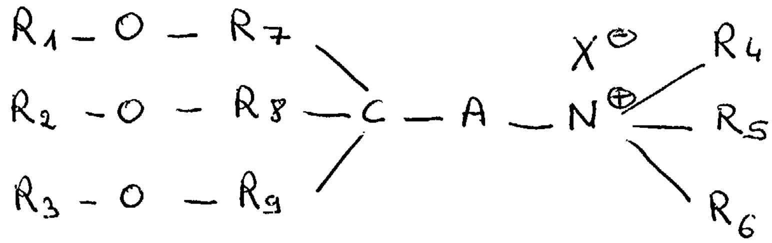

dans laquelle :

- A représente une simple liaison, un groupement NH-R', ou un groupement

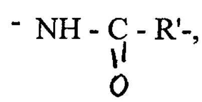 , où -R'-

  est une chaîne aliphatique comportant de 1 à 22 atomes de carbone, linéaire ou ramifiée, éventuellement substituée, saturée ou insaturée, éventuellement interrompue par un ou plusieurs des hétéroatomes O, S, N, ainsi que par un ou plusieurs radicaux carbocycliques ou hétéroycliques, saturés, insaturés ou aromatiques,

- $R_1$, $R_2$, $R_3$ sont identiques ou différents et représentent chacun un groupement alkyle ou acyle supérieur,

- $R_7$, $R_8$, $R_9$ sont identiques ou différents et représentent chacun un radical alkylène $(CH_2)_n$ où $1 \leq n \leq 6$,

- $R_4$,$R_5$,$R_6$ sont identiques ou différents et représentent chacun :

  • un atome d'hydrogène
  • un radical alkyle, alcényle, alcynyle ou acyle comportant de 1 à 22 atomes de carbone, éventuellement substitué, éventuellement interrompu par un ou plusieurs hétéroatomes choisis parmi O,S,N, ou par un ou plusieurs radicaux carbocycliques ou hétérocycliques, saturés, insaturés ou aromatiques,
  • ou encore, au moins 2 des groupements R4, R5 et R6 forment ensemble avec l'atome d'azote auquel ils

sont liés un groupement quinuclidino, piperidino, pyrrolidino ou morpholino,

- X est un anion non toxique,

pour son utilisation comme médicament.

**2.** Composé amphipathique cationique de formule I telle que définie à la revendication 1 et dans laquelle $R_7$, $R_8$ et $R_9$ sont tous identiques et représentent le groupement

- $CH_2$-, pour son utilisation comme médicament.

**3.** Composé amphipathique cationique de formule I telle que définie dans une des revendications 1 ou 2, et dans laquelle $R_4$, $R_5$ et $R_6$ sont tous identiques et représentent le groupement $CH_3$-, pour son utilisation comme médicament.

**4.** Composé amphipathique cationique de formule I telle que définie dans une des revendications 1 à 3, et dans laquelle $R_1$, $R_2$ et $R_3$ sont tous identiques et représentent un groupement acyle supérieur, pour son utilisation comme médicament.

**5.** Composé amphipathique cationique de formule I telle que définie dans une des revendications 1 à 4, et dans laquelle A représente un groupement

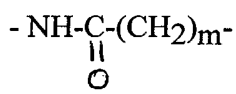

où 1≤m≤22, pour son utilisation comme médicament.

**6.** Composé amphipathique cationique de formule I telle que définie dans une des revendications 1 à 4, et dans laquelle A représente un groupement -

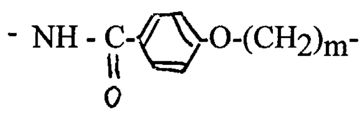

où 1 ≤m≤22, pour son utilisation comme médicament.

**7.** Composé amphipathique cationique de formule I telle que définie dans une des revendications 1 à 6, pour son utilisation comme agent de transfection.

**8.** Composé selon une des revendications précédentes, **caractérisé en ce qu'**il est associé à un lipide neutre.

**9.** Composé selon la revendications précédente, **caractérisé en ce que** le lipide neutre est la dioleoylphosphatidylethanolamine.

**10.** Composé selon une des revendications 8 ou 9, **caractérisé en ce que** le rapport molaire composé amphipathique cationique de formule I/lipide neutre est compris entre 9/1 et 1/9.

**11.** Agent de transfection, **caractérisé en ce qu'**il comprend au moins un composé selon une des revendications 7 à 10.

**12.** Agent de transfection selon la revendication 11, **caractérisé en ce qu'**il se présente sous forme de liposomes en suspension aqueuse ou lyophilisés.

**13.** Composition pharmaceutique, **caractérisée en ce qu'**elle comprend au moins un composé de formule I, telle que définie dans une des revendications 1 à 6.

**14.** Composition vaccinale, **caractérisée en ce qu'**elle comprend au moins un composé de formule I, telle que définie dans une des revendications 1 à 6.

**15.** Adjuvant pour composition vaccinale, **caractérisé en ce qu'**il comprend au moins un composé de formule I telle que définie dans une des revendications 1 à 6.

**16.** Méthode de transfection de cellules eucaryotes, **caractérisée en ce qu'**elle consiste à mélanger l'agent de transfection selon la revendication 10 ou 11 avec au moins un plasmide ou un polynucléotide, et à porter le mélange ainsi obtenu au contact des cellules à transfecter.

**17.** Utilisation d'un composé de formule I telle que définie dans une des revendications 1 à 6, pour la fabrication d'un médicament utilisable en thérapie génique.

**18.** Utilisation d'un composé de formule I telle que définie dans une des revendications 1 à 6, pour la fabrication d'un vaccin.

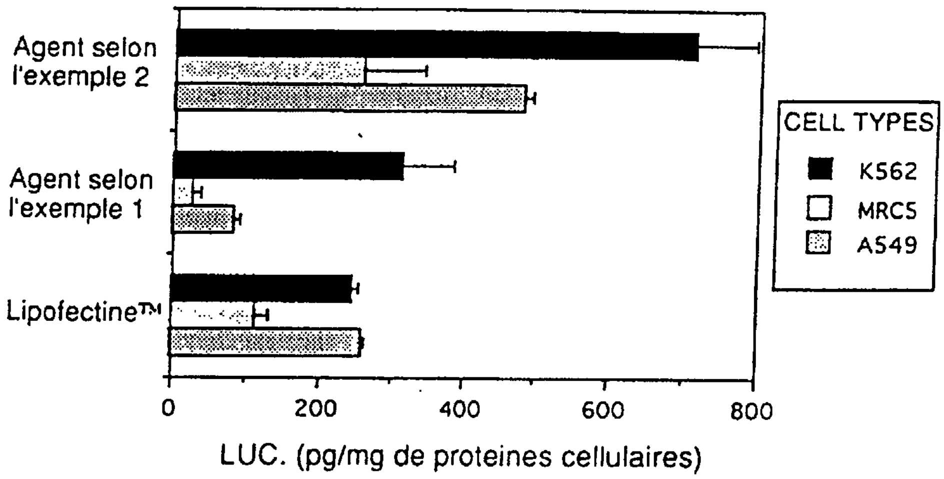
Agent selon l'exemple 2
Agent selon l'exemple 1
Lipofectine™
CELL TYPES
K562
MRC5
A549
0
200
400
600
800
LUC. (pg/mg de proteines cellulaires)

FIGURE 1

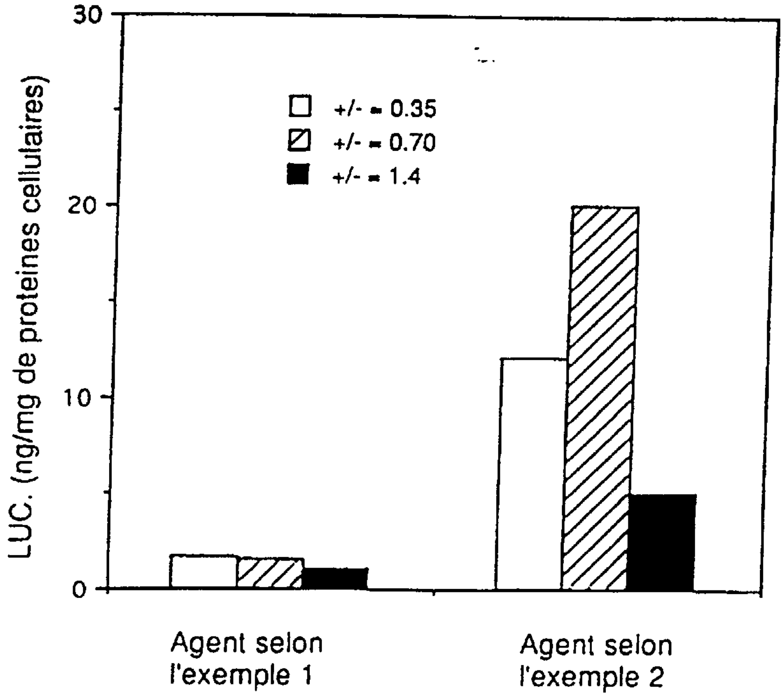
30
20
10
0
LUC. (ng/mg de proteines cellulaires)
+/- = 0.35
+/- = 0.70
+/- = 1.4
Agent selon l'exemple 1
Agent selon l'exemple 2

FIGURE 2

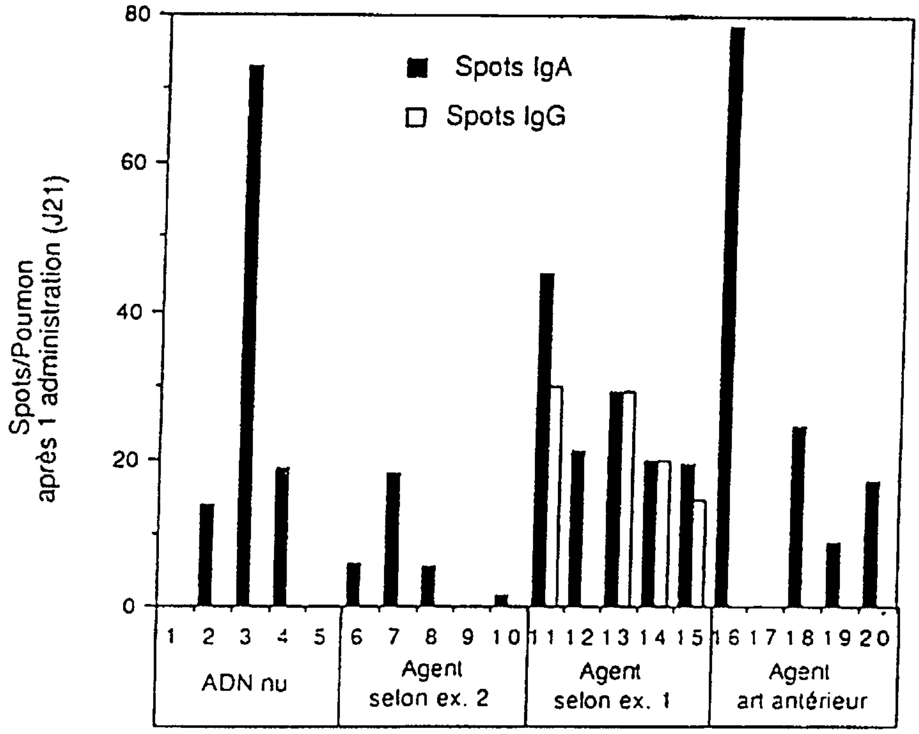
80
60
40
20
0
Spots IgA
Spots IgG
Spots/Poumon
après 1 administration (J21)
1 2 3 4 5
ADN nu
6 7 8 9 10
Agent
selon ex. 2
11 12 13 14 15
Agent
selon ex. 1
16 17 18 19 20
Agent
art antérieur

FIGURE 3

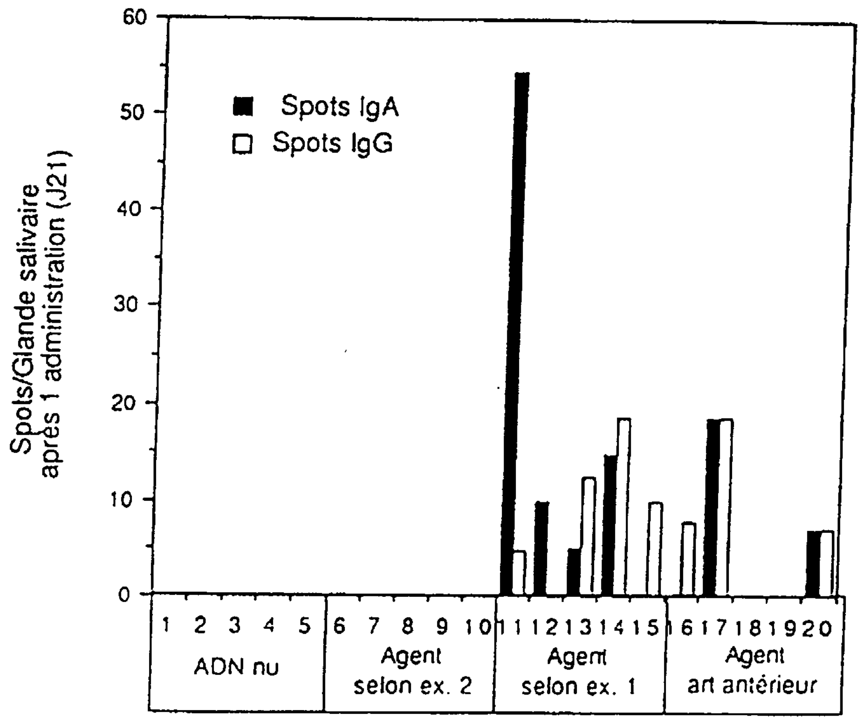
Spots IgA
Spots IgG
Spots/Glande salivaire après 1 administration (J21)
60
50
40
30
20
10
0
1 2 3 4 5
ADN nu
6 7 8 9 10
Agent selon ex. 2
11 12 13 14 15
Agent selon ex. 1
16 17 18 19 20
Agent art antérieur

FIGURE 4

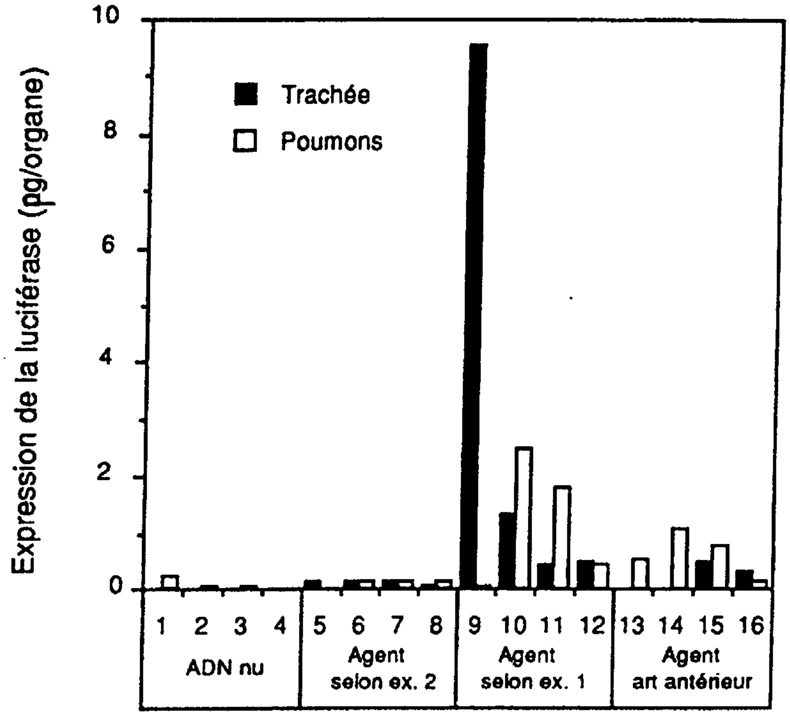
10
8
6
4
2
0
Expression de la luciférase (pg/organe)
Trachée
Poumons
1 2 3 4
ADN nu
5 6 7 8
Agent selon ex. 2
9 10 11 12
Agent selon ex. 1
13 14 15 16
Agent art antérieur

FIGURE 5